# EUROPEAN PATENT APPLICATION

(11) **EP 2 887 069 A1**
(43) Date of publication of application: **24.06.2015**
(21) Application number: 13382546.3
(22) Date of filing: 23.12.2013
(51) Int. Cl.: G01N 33/68

(54) **Methods for determining the propensity of a patient for hemorrhagic transformation after stroke**

(71) Applicant: Fundació Hospital Universitari Vall d'Hebron - Institut de Recerca, 08035 Barcelona (ES)
(72) Inventor: Merino Zamorano, Cristina, 08203 Sabadell - Barcelona (ES); Rosell Novel, Anna, 08041 Barcelona (ES); Hernández Guillamón, Mar, 08012 Barcelona (ES); Montaner Vilallonga, Joan, 08037 Barcelona (ES)
(74) Representative: ABG Patentes, S.L.

(57) **Abstract**

The invention relates to a method for predicting the risk of suffering symptomatic hemorrhagic transformation in response to the treatment with a thrombolytic agent in an ischemic stroke patient based on the determination of the level of Nurr1 in a sample of said patient. Furthermore the invention relates to a composition comprising a Nurr1 inhibitor and a thrombolytic agent and to a pharmaceutical preparation comprising the composition of the invention and a pharmaceutically acceptable excipient or carrier. The invention also relates to the use of an inhibitor of Nurr1 in the treatment of cytotoxicity associated to the treatment with a thrombolytic agent and to the use of the composition and the pharmaceutical preparation of the invention in the treatment of ischemic stroke.

SEQUENCE LISTING

<110> Fundació Hospital Universitari Vall d\222Hebron-Institut de Recerca

<120> METHODS FOR DETERMINING THE PROPENSITY OF A PATIENT FOR
HEMORRHAGIC TRANSFORMATION AFTER STROKE

<130> P9913EP00

<160> 2

<170> PatentIn version 3.5

<210> 1
<211> 19
<212> DNA
<213> Artificial Sequence

<220>
<223> sequence in the promoter for the pro-opiomelanocortin gene


<220>
<221> misc_feature
<222> (9)..(11)
<223> n is a, c, g, or t

<400> 1
tgacctttnn naaaggtca 19


<210> 2
<211> 17
<212> DNA
<213> Artificial Sequence

<220>
<223> sequence recognized by the Nurr1-RXR heterodimer


<220>
<221> misc_feature
<222> (7)..(9)
<223> n is a, c, g, or t

<400> 2
aggtcannna aaggtca 17

## Description

### Technical Field of Invention

The invention is related to the field of diagnostic methods.

### Background of Invention

Ischemic stroke is a leading cause of death and permanent disability in adults worldwide. It principally occurs because of the occlusion of a cerebral artery, typically by a thrombus, limiting the supply of oxygen and nutrients. Currently, reperfusion therapy with intravenous or intra-arterial administration of recombinant tissue plasminogen activator (r-tPA) is efficient and improves clinical outcome in human ischemic stroke. Nowadays, this is the only available treatment for acute ischemic stroke up to 4.5 h after symptom onset. Despite the high effectiveness, r-tPA is administrated to less than 10% of stroke patients, partially due to the narrow therapeutic time-window. In fact, a clear association has been found between the efficacy and the interval between onset of symptom and administration of the thrombolytic agent. The incidence of sympthomatic hemorrhagic transformation is the main adverse and more severe event after the thrombolytic treatment. Indeed, it is known that exogenous administration of r-tPA after vascular occlusion may be associated with the breakdown of the blood brain barrier (BBB), increasing endothelial permeability and brain edema specially when administered late after stroke onset. Furthermore, tPA besides being a vascular protease, it is also expressed by nerve cells and exerts important functions in the brain parenchyma. In this regard, different studies suggest that the administration of r-tPA may increase the neurotoxicity associated to ischemic stroke. Therefore, there is an urgent need to widen and improve the safety of this treatment, identifying the underlying mechanisms of r-tPA side effects. If thrombolytic complications could be predicted at the time of stroke onset as soon as the diagnosis of ischemic stroke is done, the number of patients who may benefit from early thrombolytic stroke therapy could be increased significantly. Previous studies have described the possible use of plasma biomarkers to predict hemorrhagic complications after r-tPA treatment in human ischemic strokes patients, such as matrix metalloproteinase-9 (MMP-9) (Montaner et al. Circulation. 2003; 107: 598-603; Castellanos et al., Stroke. 2007; 38: 1855-9), cellular fibronectin (Castellanos et al., Stroke. 2004;35: 1671-6; Castellanos et al., Stroke. 2007; 38: 1855-9) and vascular adhesion protein 1 (VAP-1) (Hernández-Guillamon et al., Stroke. 2010;41: 1528-35). Interestingly, in brain tissue it has been described an increase of MMP-9+ neutrophil infiltration in the infarct core areas (Justicia et. al., J Cereb Blood Flow Metab. 2003; 23:1430-40; Rosell et al., Stroke 2006, 37: 1399-1406) and this MMP-9 has been associated to collagen IV degradation in human ischemic stroke after hemorrhagic transformation episodes, relating neutrophil infiltration to BBB leakage (Rosell et al., Stroke. 2008;39(4):1121-6). Overall, these studies highlight the role of damaged endothelium after stroke related to hemorrhagic transformation and BBB breakdown under cerebral ischemia conditions. However, those markers are not yet used in clinical routine due to several reasons.

Therefore, there is a need for additional and better markers that allow predicting the propensity to suffer hemorrhagic transformation in patients who have suffered stroke and, particularly, biomarkers that can be more rapidly determined since thrombolytic treatment provides better results when applied shortly after the stroke symptoms or the stroke episode start.

### Summary of the Invention

In one aspect, the invention relates to a method for predicting the risk of suffering symptomatic hemorrhagic transformation in response to the treatment with a thrombolytic agent in an ischemic stroke patient comprising determining the level of Nurr1 in a sample of said patient, wherein elevated level of Nurr1 in said sample with respect to a reference value is indicative that the patient shows high risk of suffering symptomatic hemorrhagic transformation in response to the treatment with a thrombolytic agent.

In another aspect, the invention relates to a composition comprising a Nurr1 inhibitor and a thrombolytic agent.

In another aspect, the invention relates to a pharmaceutical preparation comprising a therapeutically effective amount of a composition of the invention and a pharmaceutically acceptable excipient or carrier.

In another aspect, the invention relates to an inhibitor of Nurr1 for use in the treatment of cytotoxicity associated to the treatment with a thrombolytic agent in a patient suffering ischemic stroke.

In another aspect, the invention relates to a composition of the invention, or a pharmaceutical preparation of the invention for use in the treatment of ischemic stroke.

In another aspect, the invention relates to a kit comprising a reagent for detecting the level of Nurr1.

In another aspect, the invention relates to the use of a kit of the invention for predicting the risk of suffering symptomatic hemorrhagic transformation in an ischemic stroke patient treated with a thrombolytic agent.

### Brief Description of the Figures

The following figures are included to further show certain aspects of the present invention. The invention may be better understood by referring to one or more of these figures in conjunction with the detailed description of specific embodiments described herein.

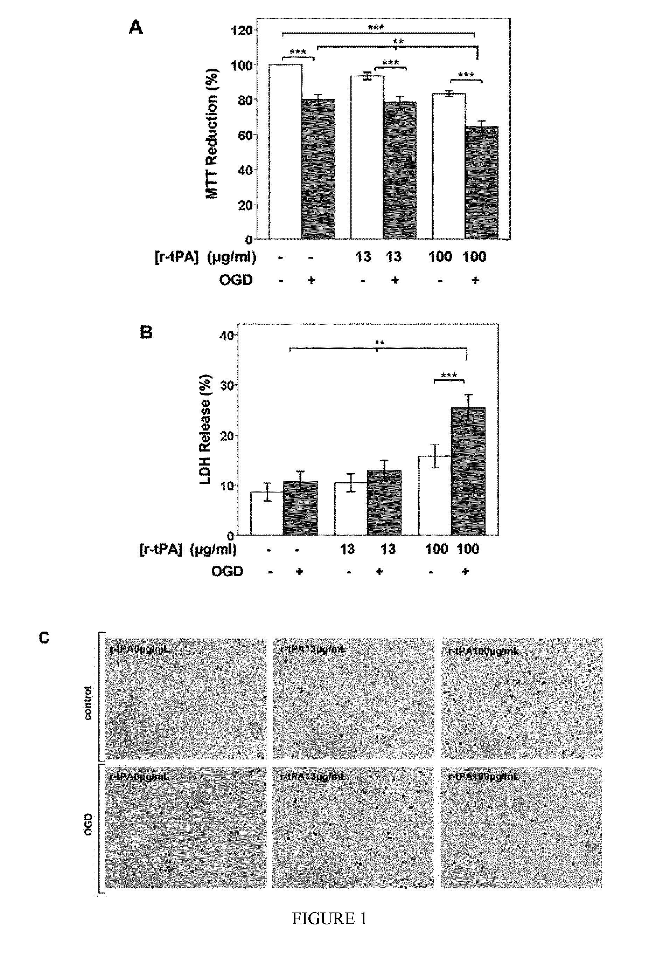

**Figure 1****. Effect of the administration of r-tPA under OGD conditions. A)** Cell viability and **B**) cell toxicity of human endothelial cells (hCMEC/D3) exposed to 6h r-tPA treatment and OGD (oxygen glucose deprivation). Results are shown as percentage of mean ± S.E.M values of at least 9 independent experiments performed in triplicate. (*** p < 0.001; **p < 0.01). C) Representative images of hCMEC/D3 cells exposed to r-tPA treatment under OGD or control conditions (normoxia/normoglycemia). 20X magnification images.

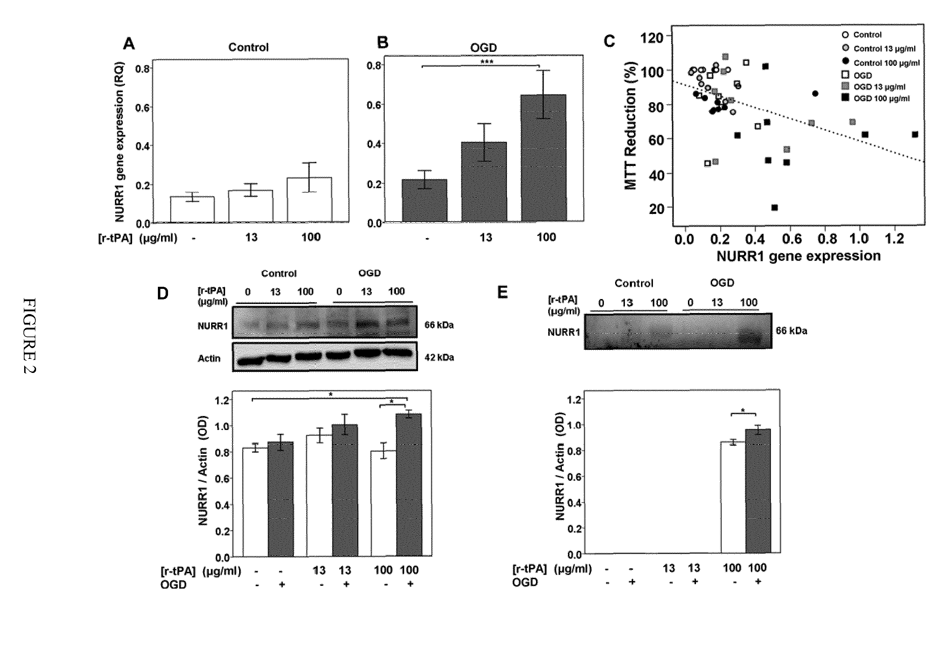

**Figure 2****. NURR1 expression in hCMEC/D3 cells. A)** PCR validation study results in control and **B**) OGD conditions **C**) Correlation between cell viability and Nurr1 gene expression. **D**) Replication study at protein level by Western Blot of cell lysates and **E**) culture supernatants. Results are shown as the mean ± S.E.M. values of at least 6 independent experiments performed in triplicate (** p < 0.01; * p<0.05).

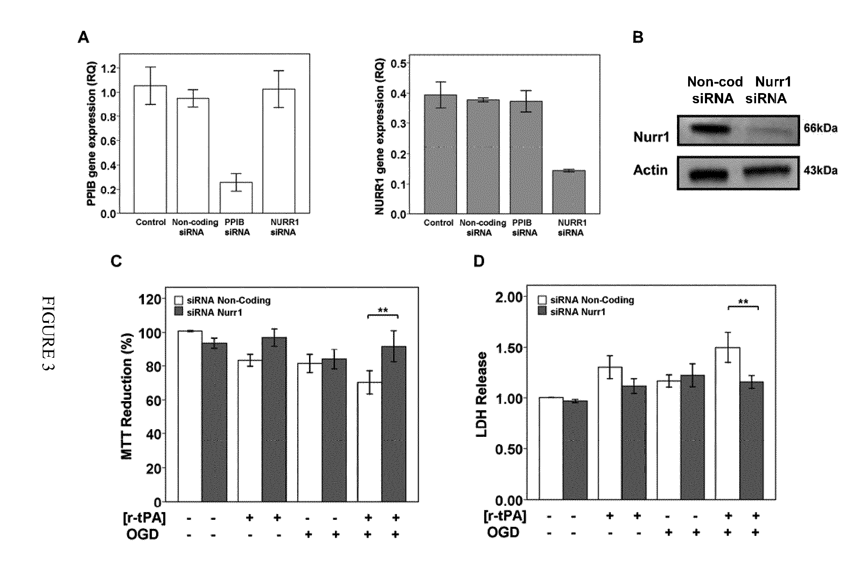

**Figure 3****. Effect of Nurr1 gene silencing in hCMEC/D3 cells. A)** PCR performed after siRNA delivery of Cyclophilin B (PPIB) and Nurr1 demonstrate the efficiency and specificity of the silencing approach. **B**) Nurr1 protein quantification in hCMEC/D3 cells by Western Blot after silencing process. **C**) Impact on cell viability and **D**) cytotoxicity after r-tPA treatment under OGD conditions in Nurr1 gene silencing cells. Results are shown as the mean ± S.E.M. of at least 6 independent experiments performed in triplicate (*p<0.05).

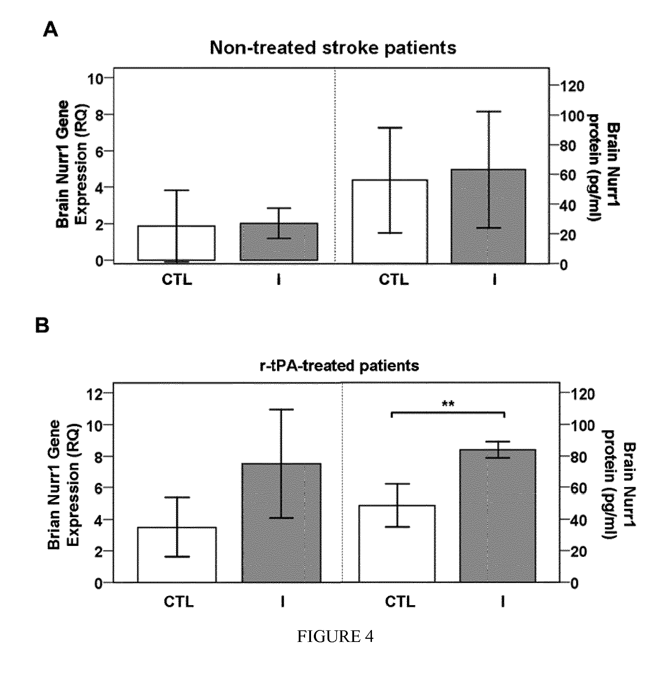

**Figure 4****. NURR1 gene and protein expression in human brain tissue. A)** Nurr1 gene and protein expression in brain of non-treated and **B**) r-tPA-treated ischemic stroke patients. N=3 in gene expression analysis and N=4 in protein expression analysis. White bars represent contralateral area (CTL) and grey bars, infarct core area (I). Results are shown as the mean ± S.D. (** p<0.01).

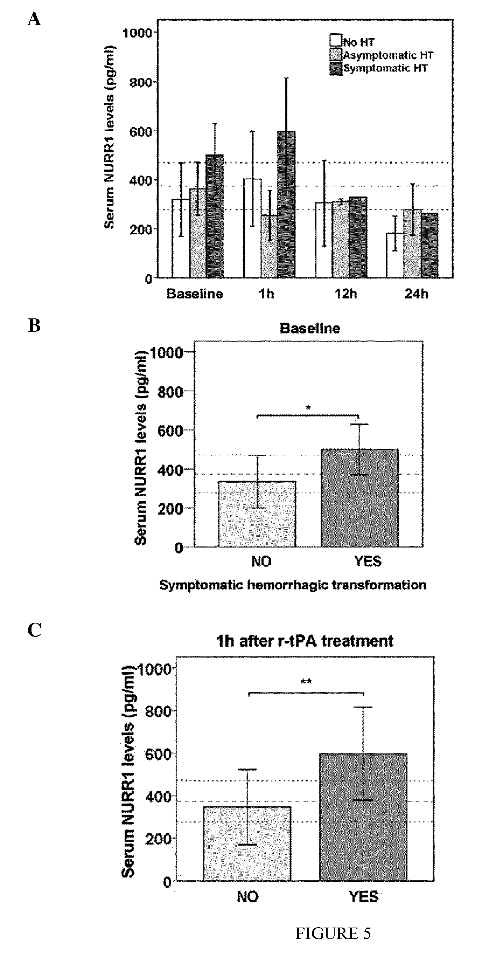

**Figure 5****. Serum NURR1 levels in r-tPA-treated ischemic stroke patients. A)** Temporal profile for NURR1 protein according to the type of HT after r-tPA treatment. **B)** Baseline NURR1 levels and (C) 1h after r-tPA treatment, according to the appearance of symptomatic HT. Dashed lines indicate the reference interval of serum Nurr1 in controls (**p<00.1; * p<0.05).

### Detailed Description of the Invention

The authors of the present invention have found that NURR1 gene expression is up-regulated by r-tPA treatment under OGD in brain capillary human endothelial cells, contributing to the enhancement of inflammatory signaling and increasing cell death. Additionally, they have detected a clinical association between Nurr1 levels and r-tPA associated hemorrhagic complications in human brain and serum of ischemic stroke patients receiving thrombolytic treatment.

Therefore, Nurr1 is a novel interesting plasma biomarker to detect hemorrhagic transformation related to thrombolytic treatment in ischemic stroke. Nurr1 regulation is a potential therapeutic target for improving r-tPA treatment safety as well.

In a first aspect, the invention relates to a method for predicting the risk of suffering symptomatic hemorrhagic transformation in response to the treatment with a thrombolytic agent in an ischemic stroke patient comprising determining the level of Nurr1 in a sample of said patient, wherein elevated level of Nurr1 in said sample with respect to a reference value is indicative that the patient shows a high risk of suffering symptomatic hemorrhagic transformation in response to the treatment with a thrombolytic agent.

As used herein, the term "prediction method" refers to a method for determining the probability that a patient suffers a hemorrhagic disorder, particularly hemorrhagic transformation. The person skilled in the art will estimate that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test as described in Dowdy and Wearden, Statistics for Research, John Wiley & Sons, New York 1983. Suitable confidence intervals are at least 50%, at least 60%, at least 70%, at least 80%, at least 90%, at least 95%. p values are, preferably, 0.2, 0.1, 0.05. Thus, the term "high probability of suffering a symptomatic hemorrhagic transformation" is understood to mean the situation where the subject shows at least 5 %, at least 10%, at least 20%, at least 30%, at least 40%, at least 50%, at least 60%, at least 70%, at least 80%, at least 90%, at least 100% probabilities of developing or suffering a symptomatic hemorrhagic transformation over time.

The term "symptomatic hemorrhagic transformation", as used herein relates to a process in stroke patients with intracranial blood extravasations showing symptoms and it is also defined as clinical deterioration temporally related to hemorrhagic transformation documented by CT-scan or autopsy. Symptomatic hemorrhage was considered to be due to study medication if it occurred within 36 hours of treatment.

In a preferred embodiment, the symptomatic hemorrhagic transformation is a parenchymal hemorrhage. The term "parenchymal hemorrhage" as used herein refers to blood clots in the infarcted area with at least slight space-occupying effect.

The term "patient", as used herein, refers to all animals classified as mammals and includes, but is not restricted to, domestic and farm animals, primates and humans, e.g., human beings, non-human primates, cows, horses, pigs, sheep, goats, dogs, cats, or rodents. Preferably, the patient is a male or female human of any age or race.

The term "ischemic stroke" refers to the physical blockage of blood flow to an area of the brain as a consequence of the occlusion of an artery, causing brain cells in the area to die. Ischemic strokes can further be divided into thrombotic and embolic strokes. Thrombotic strokes occur when a brain artery is blocked by a blood clot formed in the brain and account for approximately 50% of all strokes. Embolic strokes are caused by a thrombus, which is formed in a peripheral artery that travels to the brain where it produces ischemia

The term "thrombolytic agent" or "antithrombotic agent" is understood to mean, according to the present invention, any compound administered to the patient in a clinically effective amount able to cause thrombus breakage or clot lysis in order to restore blood circulation.

Currently available thrombolyic agents include reteplase (r-PA or Retavase), alteplase (t-PA or Activase), urokinase (Abbokinase), prourokinase, anisoylated purified streptokinase activator complex (APSAC), staphylokinase (Sak), atenecteplase (TNKasa), anistreplase (Eminase), streptoquinase (Kabikinase, Streptase) or uroquinase (Abokinase), and anticoagulant compounds, i.e., compounds that prevent coagulation and include, without limitation, vitamin K antagonists (warfarin, acenocumarol, fenprocoumon and fenidione), heparin and heparin derivatives such as low molecular weight heparins, factor Xa inhibitors such as synthetic pentasaccharides, direct thrombin inhibitors (argatroban, lepirudin, bivalirudin and ximelagatran) and antiplatelet compounds that act by inhibition of platelet aggregation and, therefore, thrombus formation and include, without limitation, cyclooxygenase inhibitors (aspirin), adenosine diphosphate receptor inhibitors (clopidrogrel and ticlopidine), phosphodiesterase inhibitors (cilostazol), glycoprotein IIB/IIIA inhibitors (Abciximab Eptifibatide, Tirofiban and Defibrotide) and adenosine uptake inhibitors (dipiridamol).

In a preferred embodiment, the thrombolytic agent is a plaminogen activator. In a yet more preferred embodiment, the plasminogen activator is tPA (tissue plasminogen activator).

The term "tissue plasminogen activator (t-PA)" as used herein refers to a serine protease found on endothelial cells that catalyzes the conversion of plasminogen to plasmin. The complete protein sequence for human t-PA has the UniProt accession number P00750 (September 9^{th}, 2013). tPA may be manufactured using recombinant biotechnology techniques, tPA created this way may be referred to as recombinant tissue plasminogen activator (rtPA). Recombinant tissue plasminogen activators (r-tPAs) include alteplase, reteplase, and tenecteplase (TNKase).

Therapeutic doses of t-PA should be given within the first 4.5 hours of the onset of symptoms. Recommended total dose: 0.9 mg/kg (maximum dose should not exceed 90 mg) infused over 60 minutes. Load with 0.09 mg/kg (10% of the 0.9 mg/kg dose) as an intravenous bolus over 1 minute, followed by 0.81 mg/kg (90% of the 0.9 mg/kg dose) as a continuous infusion over 60 minutes. Heparin should not be started for 24 hours or more after starting alteplase for stroke. Said t-PA is given intravenously and in some cases may be given directly into an artery and should be given right away after the first symptoms of stroke onset.

The first step of the method of the invention comprises determining the level of Nurr1 in a sample of the patient.

The term "sample" as used herein, relates to any sample which can be obtained from the patient. The present method can be applied to any type of biological sample from a patient, such as a biopsy sample, tissue, cell or biofluid (plasma, serum, saliva, semen, sputum, cerebral spinal fluid (CSF), tears, mucus, sweat, milk, brain extracts and the like).

In a preferred embodiment the sample is a biofluid. Illustrative non limitative biofluids are blood, plasma, serum, saliva, urine or cerebrospinal fluid. In a more preferred embodiment, the biofluid is serum.

In a preferred embodiment, the serum is obtained before the treatment with a thrombolytic agent or 1 hour after the treatment with the thrombolytic agent.

The term "Nurr1" as used herein refers to Nuclear receptor related 1 protein also known as NR4A2 (nuclear receptor subfamily 4, group A, member 2), HZF-3; NOT; RNR1 or TINUR, is a protein that in humans is encoded by the *NR4A2* gene. NURR1 is a member of the nuclear receptor family of intracellular transcription factors and plays a key role in the maintenance of the dopaminergic system of the brain. The sequence of Nurr1 protein in humans corresponds to the sequence P43354 in the UNIPROT database 24 July 2013.

Moreover, the method of the invention contemplates the use of functionally equivalent variants of Nurr1 as biomarkers in patients at risk of suffering a symptomatic hemorrhagic transformation.

The term "functionally equivalent variant" is understood to mean all those proteins derived from Nurr1 sequence by modification, insertion and/or deletion or one or more amino acids, whenever the function is substantially maintained.

The activity of the Nurr1 can be determined by measuring expression of a reporter gene operably linked to a promoter which is responsive to Nurr1, such as a promoter which includes at least one NBRE binding site. Nurr1 responsive promoters include promoters that comprise one or more NGFI-B response elements or binding sites (NBRE; 5'-AAAGGTCA-3') which Nurr1 can bind as a monomer. For example, the promoter for the osteopontin gene is a Nurr1 responsive promoter that comprises NBRE binding sites. Other promoters responsive to Nurr1 include the promoter for the tyrosine hydroxylase (TH) gene, which comprises an NBRE binding site and several NBRE-like binding sites, (Kim et al., J. Neurochem. 85: 622-634 (2003) and the promoter for the pro-opiomelanocortin gene, which comprises the palindromic nucleotide sequence 5'-TGACCTTT-N₃-AAAGGTCA-3' (SEQ ID NO:1) that Nurr1 binds as a homodimer (Maira et al., Molec. Cell. Biol. 19: 7549-7557 (1999). Nurr1 responsive promoters further include promoters which provide for NBRE-independent Nurr1 activation, for example, the promoter for the human dopamine transporter (DAT) gene, (Sacchetti et al., J. Neurochem. 76: 1565-1572 (2001), or promoters that comprise binding sites which are recognized by the Nurr1-RXR heterodimer such as the nucleotide sequence 5'-AGGTCA-N3-AAAGGTCA (SEQ ID NO:2) which is recognized by the Nurr1-RXR heterodimer in the presence of 9-cis retinoic acid (Perlmann and Jansson, Genes Dev. 9: 769-782 (1995).

Preferably, variants of Nurr1 are (i) polypeptides in which one or more amino acid residues are substituted by a preserved or non-preserved amino acid residue (preferably a preserved amino acid residue) and such substituted amino acid may be coded or not by the genetic code, (ii) polypeptides in which there is one or more modified amino acid residues, for example, residues modified by substituent bonding, (iii) polypeptides resulting from alternative processing of a similar mRNA, (iv) polypeptide fragments and/or (iv) polypeptides resulting from Nurr1 fusion or the polypeptide defined in (i) to (iii) with another polypeptide, such as a secretory leader sequence or a sequence being used for purification (for example, His tag) or for detection (for example, Sv5 epitope tag). The fragments include polypeptides generated through proteolytic cut (including multisite proteolysis) of an original sequence. The variants may be post-translationally or chemically modified. Such variants are supposed to be apparent to those skilled in the art.

As known in the art, the "similarity" between two polypeptides is determined by comparing the amino acid sequence and the substituted amino acids preserved from a polypeptide with the sequence of a second polypeptide. The variants are defined to include polypeptide sequences different from the original sequence, preferably different from the original sequence in less than 40% of residues per segment concerned, more preferably different from the original sequence in less than 25% of residues per segment concerned, more preferably different from the original sequence in less than 10% of residues per segment concerned, more preferably different from the original sequence in only a few residues per segment concerned and, at the same time, sufficiently homologous to the original sequence to preserve functionality of the original sequence. The present invention includes amino acid sequences which are at least 60%, 65%, 70%, 72%, 74%, 76%, 78%, 80%, 90%, or 95% similar or identical to the original amino acid sequence. The degree of identity between two polypeptides is determined using computer algorithms and methods which are widely known to those skilled in the art. The identity between two amino acid sequences is preferentially determined using BLASTP algorithm [BLASTManual, Altschul, S. et al., NCBI NLM NIH Bethesda, Md. 20894, Altschul, S., et al., J. Mol. Biol. 215: 403-410 (1990)].

As the person skilled in the art understands, the expression levels of Nurr1 can be determined by measuring the levels of mRNA encoded by the corresponding genes or by measuring the levels of proteins encoded by said genes, and the levels of variants thereof.

By way of a non-limiting illustration, the expression levels are determined by means of the quantification of the levels of mRNA encoded by said gene. The latter can be quantified by means of using conventional methods, for example, methods comprising the amplification of mRNA and the quantification of the amplification product of said mRNA, such as electrophoresis and staining, or alternatively, by means of Northern blot and the use of suitable probes of the mRNA of the gene of interest or of its corresponding cDNA/cRNA, mapping with the S1 nuclease, RT-PCR, hybridization, microarrays, etc. Similarly, the levels of the cDNA/cRNA corresponding to said mRNA encoded by the marker gene can also be quantified by means of using conventional techniques; in this event, the method of the invention includes a step of synthesis of the corresponding cDNA by means of reverse transcription (RT) of the corresponding mRNA followed by the synthesis (RNA polymerase) and amplification of the cRNA complementary to said cDNA. Conventional methods of quantifying the expression levels can be found, for example, in Sambrook et al., 2001 "Molecular cloning: to Laboratory Manual", 3rd ed., Cold Spring Harbor Laboratory Press, N.Y., Vol. 1-3.

In order to normalize the values of mRNA expression among the different samples, it is possible to compare the expression levels of the mRNA of interest in the test samples with the expression of a control RNA. A "control RNA" as used herein, relates to RNA whose expression levels do not change or change only in limited amounts. Preferably, the control RNA is mRNA derived from housekeeping genes and which code for proteins which are constitutively expressed and carry out essential cellular functions. Preferred housekeeping genes for use in the present invention include 18-S ribosomal protein, β-2-microglobulin, ubiquitin, cyclophilin, GAPDH, PSMB4, tubulin and β-actin.

Alternatively, it is also possible to determine the expression levels of the marker genes by means of the determination of the expression levels of the proteins encoded by said genes, since if the expression of genes is increased, an increase of the amount of corresponding protein should occur and if the expression of genes is decreased, a decrease of the amount of corresponding protein should occur.

The determination of the expression level of the protein can be carried out by immunological techniques such as ELISA, Western Blot or immunofluorescence. ELISA is based on the use of antigens or antibodies labelled with enzymes so that the conjugates formed between the target antigen and the labelled antibody results in the formation of enzymatically-active complexes. Since one of the components (the antigen or the labelled antibody) is immobilized on a support, the antibody-antigen complexes are immobilized on the support and thus, it can be detected by the addition of a substrate which is converted by the enzyme to a product which is detectable by, e.g. spectrophotometry or fluorometry. Western blot is based on the detection of proteins previously resolved by gel electrophoreses under denaturing conditions and immobilized on a membrane, generally nitrocellulose by the incubation with an antibody specific and a developing system (e.g. chemoluminiscent). The analysis by immunofluorescence requires the use of an antibody specific for the target protein for the analysis of the expression.

On the other hand, the determination of the protein expression levels can be carried out by constructing a tissue microarray (TMA) containing the subject samples assembled, and determining the levels of the protein by techniques well known in the state of the art.

Finally, the method of the invention includes the step of comparing Nurr1 levels in a patient sample with a reference value. Thus, increased levels of Nurr1 in the sample from said patient is indicative that the patient is at high risk of suffering a symptomatic hemorrhagic transformation after thrombolytic treatment. According to the present invention, Nurr1 levels are estimated to be high with respect to a reference value when the levels in the patient sample show an increase of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more.

The term "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

In a preferred embodiment, the reference value is 442.48 pg/ml.

### Composition of the invention

In another aspect, the invention relates to a composition comprising a Nurrl inhibitor and a thrombolytic agent. The term thrombolytic agent as well as typical agents have been explained previously and equally apply to this aspect of the invention.

In a preferred embodiment, the thrombolytic agent is a plasminogen activator. In a more preferred embodiment, the plasminogen activator is r-tPA.

"Nurr1 inhibitor" as used herein, relates to any compound capable of causing a decrease in the activity of Nurr1, including those compounds which prevent expression of NURR1 gene and compounds which lead to reduced Nurr1 mRNA or protein levels.

Nurrl inhibitors useful in the present invention are selected from Table 1.

| TABLE 1: NURR1 INHIBITORS SUITABLE FOR USE ACCORDING TO THE INVENTION | |
|---|---|
| I | α-synuclein (wild type and A53T variant) as described in Lin X. et al., J Neurosci 32:9248-9264 |
| II | NuIP(Nurr1-interacting protein)-specific small interfering RNA as described in Luo Y. et al., The Journal of Neuroscience, September 10, 2008 28(37):9277-9286 |
| III | P57Kip2 and PIASγ as described in Joseph et al., 2003 Proc Natl Acad Sci USA 100:15619 - 15624 and Galleguillos et al., 2004 J Biol Chem 279:2005-2011, respectively. |
| IV | Variant of Nurr1 wherein lysine 577 is substituted by arginine in the second SUMO as described in Galleguillos et al., 2004 J. Biol. Chem. 279:2005-2011. |
| V | Dominant negative Nurrl (amino acids 94-365), lacking the functional activation domain |
| VI | An interference RNA specific for the Nurrl sequence |
| VII | An antisense oligonucleotide specific for the Nurr1 sequence |
| VIII | A ribozyme or DNA enzyme specific for the Nurr1 sequence |
| IX | Inhibitory antibodies capable of binding specifically to Nurrl and inhibiting Nurr1 activity. |

As used herein, the term "interference RNA" or "iRNA" refers to RNA molecules capable of silencing the expression of NURR1 or of any gene needed for NURR1 function. To that end, iRNA are typically double-stranded oligonucleotides having at least 30 base pairs in length, and they more preferably comprise about 25, 24, 23, 22, 21, 20, 19, 18 or 17 ribonucleic acid base pairs. Several different types of molecules have been used effectively in iRNA technology including small interference RNA (siRNA) sometimes known as short interference RNA or silencer RNA, micro RNA (miRNA) which normally differ from siRNA because they are processed from single-stranded RNA precursors and they are shown to be only partially complementary to the target mRNA and short hairpin RNA (shRNA).

Small interfering RNA (siRNA) agents are capable of inhibiting target gene expression by interfering RNA. siRNAs may be chemically synthesized, or may be obtained by *in vitro* transcription, o may be synthesized *in vivo* in target cell. Typically, siRNAs consist of a double-stranded RNA from 15 to 40 nucleotides in length and may contain a protuberant region 3' and/or 5' from 1 to 6 nucleotides in length. Length of protuberant region is independent from total length of siRNA molecule. siRNAs act by post-transcriptional degradation or silencing of target messenger.

siRNA may be denominated shRNA (short hairpin RNA) characterized because the antiparallel strands that form siRNA are connected by a loop or hairpin region. siRNAs are constituted by a short antisense sequence (19 to 25 nucleotides) followed by a loop of 5-9 nucleotides, and the sense strand. shRNAs may be encoded by plasmids or virus, particularly retrovirus and, more particularly, retrovirus and under the control of promoters such as U6 promoter for RNA polymerase III.

The siRNAs of the invention are substantially homologous to NURR1 mRNA or this protein-coding genome sequence. The term "substantially honomogous" is understood to mean that siRNAs have a sequence sufficiently complementary or similar to target mRNA so that siRNA may be able to provoke mRNA degradation by RNA interference. Suitable siRNAs to provoke interference include siRNAs formed by RNA, as well as siRNAs containing chemically different modifications such as:
- siRNAs in which the links between nucleotides are different from those appearing in nature, such as phosphorothioate links.
- Stranded-RNA conjugates with a functional reagent, such as a fluorophoro.
- Modification of the ends of RNA strands, particularly the end 3' end by the combination with different functional hydroxyl groups at 2'-position.
- Sugar-modified nucleotides such as O-alkylated radicals at 2'-position such as 2'-O-methylribose or 2'-O-fluororibose.
- Base-modified nucleotides such as halogenated bases (for example 5-bromouracil and 5-iodouracil), alkylated bases (for example 7-methyl-guanosine).

The siRNAs and shRNAs of the invention may be obtained using a series of techniques known to a person skilled in the art. For example, siRNA may be chemically synthesized from protected ribonucleoside phosphoramidites in a conventional DNA/RNA synthesizer. Alternatively, siRNA may be produced by recombinant dicer from plasmid and viral vectors, where the coding region of siRNA strand or strands is under operative control of RNA polymerase III promoters. RNase Dicer processes shRNA into siRNA in cells.

The NURR1 region which is taken as a basis for the design of siRNA is not limitative and may contain a region of coding sequence (between the initiation codon and the termination codon) or, alternatively, may contain sequences from the 5' or 3' untranslated region, preferably from 25 to 50 nucleotides in length and in any position in 3' position with regard to the initiation codon. A procedure for siRNA design involves the identification of sequence motive AA(N19)TT wherein N may be any nucleotide in NURR1 sequence and the selection of those that exhibit a high content in G/C. If said sequence motive is not found, it is possible to identify sequence motive NA(N21) wherein N may be any nucleotide.

In another embodiment, the compositions of the invention comprise antisense oligonucleotides specific to NURR1, i.e., molecules whose sequence is complementary to mRNA coding for NURR1, i.e., complementary to cDNA coding chain. The antisense oligonucleotide may be complementary to a complete coding region or a region of same including both the coding region and the 5' and 3' untranslated regions. The antisense oligonucleotides may consist of 5, 10, 15, 20, 25, 30, 35, 40, 45, 50 or more nucleotides in length. The antisense oligonucleotides may be obtained by chemical synthesis or by enzymatic binding reactions widely known to a person skilled in the art. For example, an antisense oligonucleotide may further contain modified nucleotides which increase its biological stability or the stability of the bicatenary DNA-RNA complexes formed between the antisense oligonucleotide and the target polynucleotide, such as phosphorothioate derivatives, peptide nucleic acids and acridine-substituted oligonucleotides. Modified oligonucleotides that may be used for the preparation of antisense nucleic acid include 5-fluorouracil, 5-bromouracil, 5-chlorouracil, 5-iodouracil, hypoxanthine, xanthine, 4-acetyl-citosine, 5-(carboxyhydroxylmethyl) uracil, 5-carboxymethylaminomethyl-2-thiouridine, 5-carboxymethyl-aminomethyl uracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcitosine, 5-methylcitosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6- isopentenyladenine, uracil-5-oxyacetic acid, pseudouracil, queosine, 2- thiocitosine, 5-methyl-2-thiouracil, 2-thiouracil, 4-thiouracil, 5-methyluracil, uracil-5-oxyacetic acid methyl ester, 5-methyl-2-thiouracil, 3-(3-amino-3-N-2- carboxypropyl)uracil, and 2,6-diaminopurine. Alternatively, the antisense nucleic acid may be produced biologically using an expression vector in which the antisense-oriented nucleic acid has been cloned.

Another group of compounds that may form part of the compositions of the present invention are catalytically active nucleic acids known as ribozimes. Ribozimes comprise a catalytic region and a second region whose sequence is complementary to target nucleic acid and confers substrate specificity on the ribozime. After the interaction between the ribozime and its substrate by hybridization and coupling between complementary regions of target nucleic acid and ribozime, an activation of the catalytic region is produced provoking the inter- or intramolecular rupture of target nucleic acid. Basic considerations for the design of ribozimes are widely known to a person skilled in the art (see, for example, Doherty and Doudna (Annu. Ref. Biophys. Biomolstruct. 2000; 30:457- 75).

Another type of compounds that may form part of the compositions of the invention includes inhibitory antibodies. The term "inhibitory antibody" is understood to mean, according to the present invention, an antibody that is capable of binding to NURR1 provoking the inhibition of its activity. Antibodies may be prepared using any method known to a person skilled in the art. Thus, polyclonal antibodies are prepared by immunization of an animal with the protein aimed to be inhibited. Monoclonal antibodies are prepared using the method described by Kohler, Milstein et al (Nature, 1975, 256: 495). Once antibodies capable of binding to Nurrl are identified, those antibodies capable of inhibiting Nurrl activity using the abovementioned assays for determination of Nurrl activity will be selected. Suitable antibodies in the present invention include intact antibodies which comprise an antigen-binding variable region and a constant region, fragments "Fab", "F(ab')2" y "Fab"', Fv, scfv, diabodies and bispecific antibodies.

Other compounds capable of inhibiting NURR1 expression that may form part of the compositions of the invention include aptamers and spiegelmers. Aptamers and spiegelmers are single-stranded or double-stranded D- or L-nucleic acids that specifically bind to the protein resulting in a modification of the biological activity of protein. Aptamers and spiegelmers are 15 to 80 nucleotides in length and, preferably, 20 to 50 nucleotides in length.

In a preferred embodiment, the Nurrl inhibitor is a compound capable of inhibiting Nurr1 in the endothelium selectively.

In addition, the combination therapy containing a Nurrl inhibitor and a thrombolytic agent may be complemented with agents that inhibit other factors related to hemorrhagic transformation, for example matrix metalloproteinase (MMP-9), whose serum levels are high under conditions in which brain hemorrhage occurs after treatment with thrombolytic agents. Thus, the invention contemplates the combined use of NURR1 inhibitors as above described, thrombolytic agents and MMP-9 inhibitors. In a preferred embodiment, MMP-9 inhibitor is BB-3103, Ro 31-9790, BB-1101, BB-2293, broad-spectrum MMP inhibitor GM-6001 (also denominated Galardin and Ilomastat), selective gelatinase inhibitor SB-3CT, as well as the agents described by Nakatani et al (Bioorganic & Medicinal Chemistry, 2006, 14:5402-5422), Mackarel et al (Am. J. Respir. Cell Mol. Biol., 1999, 20:1209-1219) and Yang et al. (J. Cerebral Blood Flow & Metabolism, 2007, 27:697-709), whose whole content is incorporated in the present invention.

VAP-1 levels are known to be significantly higher in patients who experienced hemorrhagic transformation (see Hernandez-Guillamón M. et al, Stroke. 2010 Jul;41(7):1528-35). Thus, the invention contemplates the combined use of Nurrl inhibitors as above describe, thrombolytic agents and Vap-1 inhibitors. In a preferred embodiment, the Vap-1 inhibitor is semicarbazide.

These additional agents may form part of the pharmaceutical composition or, alternatively, may be formulated as a separate pharmaceutical composition for its administration at the same time (simultaneous administration) as the pharmaceutical compositions of the invention or at different times (sequential administration) with regard to the pharmaceutical composition provided by this invention.

The therapeutic compositions of the present invention may be used for their combined, sequential or separate use with drugs that are useful in the treatment of acute ischemic stroke, such as the neuroprotective drugs, minocycline, citiholine, albumin, as well as antiinflammatory drugs and agents that avoid infiltration of neutrophils into brain parenchyma, as simvastatin, interferon-beta and the like.

For their medicinal use, the compositions of the invention constituted by a NURR1 inhibitor and a thrombolytic agent may be found in a pharmaceutical composition.

Thus, in a further aspect, the invention relates to a pharmaceutical preparation comprising a therapeutically effective amount of a composition according to the invention and a pharmaceutically acceptable excipient or carrier.

The term "therapeutically effective amount" is understood to mean, according to the present invention, the amount of a Nurrl inhibitor sufficient to provoke a delay in hemorrhagic transformation occurrence in response to treatment of stroke with thrombolytic agents.

The combination of compounds of the invention may be found as a prodrug, salt, solvate or clatrate, whether in an isolated dosage form or in combination with additional active agents.

Preferred excipients to be used in the present invention include sugars, starches, celluloses, gums and proteins. In a particular embodiment, the pharmaceutical composition of the invention will be formulated as a solid pharmaceutical dosage form (for example tablets, capsules, coated tablets, granules, suppositories, sterile crystalline or amorphous solids that can be reconstituted to provide liquid forms, etc.), liquid dosage form (for example solutions, suspensions, emulsions, elixirs, lotions, ointments, etc.) or semisolid dosage form (gels, pomades, creams and the like). Examples of pharmaceutically acceptable carriers are known in prior art and include phosphate buffered saline solutions, water, emulsions, such as oil/water emulsions, different types of humectants, sterile solutions, etc.

The pharmaceutical compositions of the invention may be administered by any route, including, without limitation, oral, intravenous, intramuscular, intraarterial, intramedullary, intrathecal, intraventricular, transdermal, subcutanous, intraperitoneal, intranasal, enteric, topical, sublingual or rectal route. A review of the different dosage forms of active ingredients and excipients to be used and their manufacturing processes is provided in "Tratado de Farmacia Galénica", C. Faulí and Trillo, Luzán 5, S.A. de Ediciones, 1993 and in Remington's Pharmaceutical Sciences (A.R. Gennaro, Ed.), 20th edition, Williams & Wilkins PA, USA (2000).

In the particular case that the Nurr1 inhibitor is a nucleic acid (siRNA, antisense oligonucleotides, ribozimes, aptamers and spiegelmers), the pharmaceutical compositions may be formulated as a composition intended for use in gene therapy; by way of illustration and not limitation, the pharmaceutical composition of the invention may contain a viral or nonviral vector, which comprises a polynucleotide of the invention or a gene construction of the invention. By way of illustration and not limitation, said vectors, may be viral, for example, based on retrovirus, adenovirus, etc., or nonviral such as ADN-liposome, ADN-polymer, ADN-polymer-liposome complexes, etc. [see "Nonviral Vectors for Gene Therapy", edited by Huang, Hung and Wagner, Academic Press (1999)]. Said vectors, which contain a polynucleotide or a gene construction of the invention, may be administered directly to humans or animals by conventional methods. Alternatively, said vectors may be used to transform, or transfect or infect cells, for example, mammal cells, including human, *ex vivo,* which subsequently will be implanted into a human body or an animal to obtain the desired therapeutic effect. For administration to a human body or an animal, said cells will be formulated in a suitable medium that will have no adverse influence on cell viability.

### Therapeutic uses of the invention

In another aspect, the invention relates to an inhibitor of Nurrl for use in the treatment of cytotoxicity associated to the treatment with a thrombolytic agent in a patient suffering ischemic stroke.

In a preferred embodiment, the thrombolytic agent is a plasminogen activator. In a more preferred embodiment, the plasminogen activator is r-tPA.

In another preferred embodiment, the Nurrl inhibitor is selected from the compound of Table 1.

The terms Nurrl inhibitor, patient, ischemic stroke and thrombolytic agent have been explained previously and equally apply to this aspect of the invention

The term "cytotoxicity" as used herein refers to the destruction of living cells.

The person skilled in the art knows several assays suitable for detecting cytotoxicity in a cell, for example assessing cell membrane integrity using dyes, such as trypan blue or propidium iodide. Alternatively, membrane integrity can be assessed by monitoring the passage of substances that are normally sequestered inside cells to the outside. One molecule, lactate dehydrogenase (LDH), is commonly measured using LDH assay. Cytotoxicity can also be monitored using the 3-(4, 5-Dimethyl-2-thiazolyl)-2, 5-diphenyl-2H-tetrazolium bromide (MTT) or MTS assay or using resazurin. Cytotoxicity can also be measured by the sulforhodamine B (SRB) assay, WST assay and clonogenic assay.

In another aspect, the invention related to a composition of the invention or a pharmaceutical preparation of the invention for use in the treatment of ischemic stroke.

Dosage regimen will be determined by the physician and the clinical factors. As it is well known in medicine, dosage depends on several factors that include the physical features of the patient (age, size, sex), administration route, severity of disease, compound to be used and pharmacokinetic properties of the subject.

### Kit of the invention

In another aspect, the invention relates to a kit comprising a reagent for detecting the level of Nurr1.

The term "kit", as used herein, refers to a product containing the different reagents necessary for carrying out the methods of the invention packed so as to allow their transport and storage. Materials suitable for packing the components of the kit include crystal, plastic (e.g. polyethylene, polypropylene, polycarbonate), bottles, vials, paper, or envelopes.

Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instructions.

The reagents of the kit include compounds that bind specifically to the marker protein. Preferably, said compounds are antibodies, aptamers or fragments thereof.

In a preferred embodiment, the reagent is an antibody or fragments thereof.

The antibodies of the kit of the invention can be used according to techniques known in art for determining the protein expression levels, such as, for example, flow cytometry, Western blot, ELISA, RIA, competitive EIA, DAS-ELISA, techniques based on the use of biochips, protein microarrays, or assays of colloidal precipitation in reactive strips.

The antibodies can be fixed to a solid support such as a membrane, a plastic or a glass, optionally treated to facilitate the fixation of said antibodies to the support. Said solid support comprises, at least, a set of antibodies which specifically recognize the marker, and which can be used for detecting the levels of expression of said marker.

Additionally, the kit of the invention comprises reagents for detecting a protein encoded by a constitutive gene. The availability of said additional reagents allows normalizing the measurements performed in different samples (for example, the sample to be analyzed and the control sample) to rule out that the differences in the expression of the biomarkers are due to a different quantity of total protein amount in the sample more than the real differences in the relative levels of expression. The constitutive genes in the present invention are genes that are always active or being transcribed constantly and which encode for proteins that are expressed constitutively and carry out essential cellular functions. Proteins that are expressed constitutively and can be used in the present invention include, without limitation, β-2-microglobulin (B2M), ubiquitin , 18-S ribosomal protein, cyclophilin, GAPDH, PSMB4, tubulin and actin

In a preferred embodiment, the reagents for assaying the levels of the different biomarkers comprise at least 10%, at least 20%, at least 30%, at least 40%, at least 50%, at least 60%, at least 70%, at least 80%, at least 90% or at least 100% of the total amount of reagents for assaying biomarkers forming the kit. Thus, in the particular case of kits comprising reagents for assaying the levels of Nurr1, the reagents specific for said biomarkers (i.e. antibodies which bind specifically to Nurr1) comprise at least 10%, at least 20%, at least 30%, at least 40%, at least 50%, at least 60%, at least 70%, at least 80%, at least 90% or at least 100% of the antibodies present in the kit.

In another aspect, the invention relates to the use of the kit of the invention for predicting the risk of suffering symptomatic hemorrhagic transformation in an ischemic stroke patient in response to a thrombolytic agent.

The invention will be described by way of the following examples which are to be considered as merely illustrative and not limitative of the scope of the invention.

### EXAMPLES

### Materials and methods

### Preparation of hCMEC/D3 cell line cultures

Human cerebral microvascular endothelial cells (hCMEC/D3) were provided by P.O. Couraud, Cochin Institute, Paris, France (Weksler, 2005). Cells were grown in EGM-2 medium (Lonza, Barcelona, Spain) supplemented with VEGF, insulin-like growth factor, bovine FGF, hydrocortisone, ascorbate, and 2% fetal bovine serum (FBS) (Lonza). Cultures were kept at 37 °C in a humidified incubator containing 5% CO₂ and atmospheric oxygen. If not specified, cells were seeded at 100.000 cells/ml in EGM-2 medium in 6 or 24 well plates, previously pre-coated with collagen I (1:50) (Rat Collagen I, Cultrex; Trevigen, Gaithersburg, MD, USA).

### Oxygen and glucose deprivation (OGD) and r-tPA treatment

Eighty percent confluent cultures were exposed to OGD by placing them in an anaerobic chamber (Invivo2, Ruskinn, Pencoed, UK), containing 0,5 % O₂, 4,5% CO₂, 95% N₂ at 37°C for 6 h with deoxygenated glucose-free medium (RPMI) (Gibco, Invitrogen, Madrid, Spain). Normoxic/Normoglycemic cultures (control condition) were kept at 37°C in a humidified incubator containing 5% CO₂ and atmospheric oxygen with RPMI with glucose medium (Sigma-Aldrich, Madrid, Spain). Cultures were treated with 13 or 100 µg/ml of r-tPA (Actilyse®, Boehringer, Ingelhem, Germany) or distillated water under OGD or control conditions. After treatments, RNA was extracted from cell lysates using RNeasy® Mini Kit (Qiagen, Valencia, CA, USA), following the manufacturer's instructions and stored at -80°C until needed. Cell culture supernatants were collected in a 2 ml tubes and centrifuged at 8000 rpm at 4°C for 10 min to eliminate cellular rests and supernatants were transferred to a new sterile 2 ml tubes and stored at -80 C°. Cell culture lysates were collected using a lysis buffer (50mM Tris-HC ph7.6, 150mM NaCl, 5mM CaCl₂, 0.05% BRI-35, 0.02% NaN₃ and 1% Triton X-100), containing 1% phenylmethylsulfonyl fluoride (PMSF, Sigma Aldrich) and 0.5% Aprotinin from Bovine Lung (Sigma-Aldrich), and stored at -80C° until ready to use.

### Cell viability and toxicity assays

Cell viability was determined by MTT (3-(4,5-dimethylthiazol-2-yl)-2,5-diphenyltetrazolium bromide; Sigma-Aldrich) reduction. After treatments, 0.5 mg/ml MTT was added to cells and kept at 37°C during 90 min. Then, the medium was replaced by dimethyl sulfoxide (DMSO). The amount of formazan blue formed after MTT reduction was quantified spectrophotometrically at 575 nm. Results were expressed as the percentage of viable cells compared to non-treated controls for each experiment. Lactate dehydrogenase (LDH) released from damaged cells was used to determine cell cytotoxicity. LDH activity was analyzed in culture media and culture lysates following the manufacturer's instructions (Roche Applied Science, Indianapolis, IN, USA). Spectrophotometric measurements were determined at 490nm. LDH release was expressed as percentage of total cell LDH and plotted as percentage of LDH release after treatments.

### RNA purification and hybridization for Microarrays gene expression analysis

RNA was extracted from hCMEC/D3 samples using the RNeasy kit (Qiagen, Austin, TX, USA) following manufacturer's instructions. Then, quality was assessed using the Bioanalyzer Nanochip platform (Agilent, CA, USA). Only RNA with a RIN (RNA integrity number) ≥ 5 was used for the RNA hybridization. An additional cycle of cDNA synthesizer and amplification was performed to all samples. Briefly, cDNA was synthesized from total purified RNA and biotin-labeled complementary cRNA was synthesized from cDNA. A microarray study was performed to carry out a genome-wide comparison, in order to identify differentially expressed genes using an Adjusted p-value < 0.05 cutoff induced by the r-tPA effect in control or OGD conditions. Gene expression profiles were obtained using 30 GeneChip® Human Genome U133 Arrays (Affymetrix, Santa Clara, CA, USA) in Cartridge Format (analysis of 47,000 transcripts). All samples demonstrated characteristics of high-quality cRNA and were subjected to subsequent analysis. Raw expression values obtained directly from. CEL files were preprocessed using the RMA (Robust Multi-Array) method, a three-step process which integrates background correction, normalization and summarization of probe values. These normalized values were the basis for all the analyses. The selection of differentially expressed genes between conditions was based on a linear model analysis with empirical Bayes moderation of the variance estimates. P-values were adjusted to obtain strong control over the false discovery rate (FDR) using the Benjamini and Hochberg method. Array quality was measured by a series of parameters which are usually provided by the core facility to confirm that the arrays had been properly hybridized. To confirm the data quality, standard approaches for quality control were conducted (standard quality controls based on Affymetrix original methods and Probe-level models) plus background correction, normalization and filtering. All the statistical analyses were done using the free statistical language R and the libraries developed for microarray data analysis by the Bioconductor Project (www.bioconductor.org). Statistical and bioinformatics analysis were performed by the Statistics and Bioinformatics Unit (UEB) of the Research Institut of Vall d'Hebron. A functional analysis to identify the biological mechanisms, pathways and functions and the most relevant genes was performed using the Ingenuity Pathways Analysis (IPA) software and database (www.ingenuity.com). Briefly, the dataset containing symbol genes and corresponding fold changes and Adj-p values was uploaded into the web-delivered application and each gene identifier was mapped to its corresponding gene object.

### Quantitative Real Time Polymerase chain reaction (qPCR): gene expression analysis

RNA concentration and quality were measured with NanoChips using the Bioanalizer 2100 system (Agilent, Santa Clara, CA, USA) or/and using spectrophotometer NanoDrop ND-1000 (NanoDrop, Wilmington, DE, USA), and corresponding cDNA was synthesized using the High-Capacity cDNA Archive kit (Applied Biosystems, Foster City, CA). mRNA levels were quantitated by real-time PCR using TaqMan fluorogenic probes (Applied Biosystems). qPCR reactions were run in triplicate and analyzed using Applied Biosystems SDS 7900 system software (Applied Biosystems). Results were normalized to glyceraldehyde-3-phosphate dehydrogenase (GAPDH, Hs99999905_ml) or PPIA (Hs99999904_ml) expression levels to account for variability in the initial sample concentration. Data were expressed as relative quantification, calculating the fold change expression values with respect to the average expression of a calibrator sample. The RQ values were calculated by use of the Livak equation: RQ = 22DDCt and the results are expressed as a ratio depending on the calibrator sample used in all experiments.

The differential expression of the selected genes from hCMEC/D3 cells challenged to OGD and/or r-tPA significantly altered in the microarray analysis was replicated by RT-qPCR using RNA samples from new cultures submitted to OGD/r-tPA treatment. From the microarray results, the candidate gene selected was NR4A2 (Hs00428691_m1)

### Western Blot (WB) Analysis

Briefly, 15 µg of whole proteins of cell culture lysates or 30 µl of the respective cell culture supernatants were separated on 10 or 12% acrilamide SDS-PAGE under reducing conditions and transferred onto 0.45-µm pore size polyvinylidene difluoride membranes (Millipore Corp., Billerica, MA, USA). Membranes were subsequently blocked for 1 h with 10% nonfat milk in PBS containing 0.1% Tween 20 (PBS-T), and probed with anti-NURR1, 1:500 (PP-N1404-00, R&D systems Inc.) diluted in 10 % nonfat milk in PBS-T overnight at 4°C. After thorough washes with PBS-T, membranes were immunoreacted with HRP-conjugated anti-mouse IgG (NA931, GE Healthcare Bio-Sciences Corp., Piscataway, NJ USA, 1:1000) for 1 h at room temperature and further developed by enhanced chemiluminescence using Supersignal West Pico Luminol/Enhancer and Stable Peroxide solutions (Thermo Scientific, Rockford, IL, USA). Anti-β actin (βActin, 1:10.000; Sigma-Aldrich) was used as loading control. The amount of target protein of different cultures was normalized with a calibrator sample, included in each gel. Images were analyzed with ImageJ software (rsbweb.nih.gov).

### Gene Silencing

Gene silencing of NR4A2/Nurr1 was performed through passive siRNA delivery via Accell SMART pool siRNA (Thermo Scientific-Dharmacon, Rockford, IL, USA), following the manufacturers protocol. Briefly, cells plated at 80,000 cells/ml and seeded in 96 or 24-well, were treated separately with the respective siRNA solutions constituted by each siRNA pool at final concentration of 1µM. Non-targeting/Non-Coding siRNA was employed as a negative control to measure the minimal toxicity and side effects of the assay. PPIB siRNA was used as positive controls to test the efficiency and specificity of NURR1 silencing, using the endogenous control PPIA (Hs99999904_ml, Applied Biosystems). To assess the effect of NURR1 knockdown on r-tPA-induced toxicity under OGD conditions, cells were pre-incubated 48 h in the siRNA-containing Accell media and subsequently replaced by RPMI media with r-tPA at 0 or 100 µg/ml and exposed to OGD or control conditions. After exposition, RNA was collected and cDNA was synthesized as previously described.

### TaqMan® Array Human Immune Panel

cDNAs from silenced cultures were mixed with equal volumes of TaqMan 2X PCR master mix from one-step RT-qPCR kit (Applied Biosystems) and 300-400 ng of cDNAs were loaded per port of the TaqMan Human Immune panel (Applied Biosystems, U.K.). This panel includes the analysis of the expression of 90 genes, including cytokines, chemokines, growth factors, immune regulators, apoptosis markers, ischemia markers, tissue-specific markers, and 6 classic endogenous controls. The plates were run on a 7900HT Fast Real-Time PCR system (Applied Biosystems, U.K.). Relative gene expression values were obtained employing comparative Ct method (described above) using cultures treated with Non-coding siRNA processed as calibrators. GAPDH, ACTB, TFRC, GUSB and PGK1 genes were used as a endogenous controls.

### Human ischemic stroke brains

Brain samples were obtained from 8 deceased patients who suffered an ischemic stroke within the previous 5 days; 4 of them were treated with r-tPA (Actilyse®, Boehringer) within the first 4.5 hours of symptom onset. All of them suffered a symptomatic hemorrhagic transformation. On autopsy and during macroscopic examination, core areas were identified by an experienced neuropathologist. From stroke brain patients, core and contralateral grey/white matter areas were obtained within the first 15 h after death, snap frozen in liquid nitrogen and stored at -80C° until RNA isolation or tissue homogenates preparation was performed (Rosell et al., 2006). NURR1 gene expression and protein levels in r-tPA-treated and non-treated patients were quantified by RT-qPCR and ELISA (see below). Demographic and tissue sampling data are listed in Table 2.

**Table 2. Demographic features of ischemic stroke patients. MCAO = Middle cerebral**

| **Case nº** | **Age (years)** | **Sex** | **Cause of death** | **Stroke Ethiology** | **r**-**tPA** | **HT** | **Evolution time (h)** | **Vascular risk factors** | **Brain Study** |
|---|---|---|---|---|---|---|---|---|---|
| 1 | 77 | female | Left MCAO + HT | Probable CE | + | + | 72 | AF, HTN | qPCR, WB |
| 2 | 83 | female | Left MCAO + HT | CE | + | + | 100 | AF | WB |
| 3 | 73 | female | Left MCAO + HT | Undetermined | + | + | 44 | DLP | qPCR, WB |
| 4 | 75 | male | Left MCAO + HT | Undetermined | + | + | 19 | HTN | qPCR, WB |
| 5 | 60 | male | Left MCAO + HT | Dissection | - | + | 120 | HTN | WB |
| 6 | 72 | male | Right MCAO + HT | Atherothrombotic | - | + | 13 | HTN | qPCR, WB |
| 7 | 79 | female | Left MCAO + HT | Atherothrombotic | - | + | 88 | HTN | qPCR, WB |
| 8 | 80 | male | Right MCAO + HT | Pro bable CE | - | + | 100 | HTN, DLP, AF | qPCR, WB |

| | | | | | | | | | |
|---|---|---|---|---|---|---|---|---|---|
| artery occlusion; CE = cardioembolic; HT = Hemorrhagic transformation; AF = atrial fibrillation; HTN = Hypertensive; DLP = Dyslipidemia; AMI = Acute Myocardial infarction. | | | | | | | | | |

### Human serum from ischemic stroke patients

Peripheral blood samples were obtained from ischemic stroke patients at baseline (before r-tPA administration), 1 h (by the end of r-tPA infusion), 12 h and 24 h after stroke onset. Patients received r-tPA (Actilyse®, Boehringer) in a standard 0.9-mg/kg dose. A detailed history of vascular risk factors was obtained from each patient. Our cohort included 28 stroke r-tPA treated strokes and 9 controls (free stroke), matched by sex and age. Clinical examination was performed on admission and at 12, 24, and 48 hour after symptom onset. Stroke severity was assessed based on the National Institutes of Health Stroke Scale. We defined neurological improvement as a decrease in National Institutes of Health Stroke Scale score by ≥ 4 points and defined neurological deterioration as either death or an increase in this by ≤ 4 points at 24 or 48 h. All patients underwent Computed Tomography (CT) examination within the first 3 h of stroke onset; this was repeated after 24 to 48 h (or earlier in the case of rapid neurological deterioration) to evaluate the presence of hemorrhagic transformation (HT). The CT scans were reviewed by a neuroradiologist with extensive experience in acute stroke. The presence and HT type were defined according to previously published criteria (Hacke et al., 1995); hemorrhagic infarction (HI) was defined as a petechial infarction without a space-occupying effect, and parenchymal hemorrhage (PH) was defined as hemorrhage with a mass effect. CT-based HT subtypes were defined as: HI-1, for small petechiae along the margins of the infarct; HI-2, for more confluent petechiae within the infracted area; PH-1, when hematoma involved >30% of the infarcted area with some slight space-occupying effect; PH-2, when hematoma involved <30% of the infarcted area with substantial mass effect; or PH-R, when the clot was remote from the infarcted area. Symptomatic intracranial hemorrhage was defined as blood at any site in the brain on the CT scan and documentation of neurological deterioration. To study whether serum NURR1 levels were associated to r-tPA treatment complications, patients were selected according to the presence of HT after receiving r-tPA. Thus, 50% of the patients suffered HT, and among them, 6 patients suffered a symptomatic HT.

Samples were collected in tubes (without any anticoagulant agent), and immediately were processed by centrifugation at 3500 rpm for 15 min at 4°C to obtain serum and aliquots were stored at -80°C until analysis was performed. The data sampling is listed in supplemental data 2. Our study was approved by the Ethics Committee of Vall d'Hebron Hospital (Barcelona, Spain), and all patients or their relatives gave written informed consent.

### NURR1 Enzyme Linked ImmunoSorbentAssay (ELISA)

ELISA kit from Cusabio (Cusabio Biotech, Wuhan, China) was used for quantitative determinations of NURR1 protein level according to manufacturer's instructions. For tissue brain samples, total protein content of brain homogenates was determined by the BCA method and equal amounts, 200 µg, were assayed. For serum samples, 50 µl was analyzed in duplicate. The mean intra-assay coefficient of variation of protein contents was lower than 25% for all samples measured.

### Statistical analysis

SPSS 15.0 package was used for statistical analyses and figures plotting bar graphs. Kolmogorov-Smirnov test was used to verify normality. Statistical significance for intergroup differences was assessed by the Mann-Whitney U and Kruskal-Wallis tests in non-parametric cases and by the T-test or One-way ANOVA analysis with Bonferroni Multiple comparisons post-hoc test in normal variables. Correlations between numerical variables were assessed by Pearson's or Spearman correlation coefficient as appropriate. Receiver operator characteristic (ROC) curves were used to calculate the sensitivity and specificity of Nurr1 levels for predicting symptomatic HT in r-tPA treated stroke patients. A p value ≤0.05 was considered statistically significant at a 95% confidence level.

### Results

### Example 1

### Gene expression profile induced by r-tPA under OGD in brain endotelial cells

Six hours treatment of 13 µg/ml r-tPA, which is translatable to the r-tPA plasma concentration found in acute ischemic stroke patients receiving thrombolytic therapy, was not toxic to cultured cerebral endothelial cells, whereas 100 µg/ml r-tPA induced a significant cell viability reduction. OGD triggered a higher reduction and the combination of OGD and r-tPA treatment showed a summative decrease of cell viability in a r-tPA dose-dependent manner, which was associated to a significant increase in cell toxicity compared to the corresponding control and non-treated condition (Figure 1). A microarray study was performed in order to carry out a genome-wide comparison of brain endothelial cells treated with both 13 and 100 µg/ml concentrations of r-tPA under OGD. A total of 2500 analyzed transcripts (Adj-p<0.05) were regulated by 6 h OGD. Among them, 299 genes were differentially expressed only by OGD without r-tPA treatment, and 767 genes were regulated by OGD independently of the r-tPA dosage.

In order to specifically study the effect of r-tPA under OGD, genes the expression of which was affected by OGD without r-tPA were excluded from the analysis.

When the microarray analysis results were validated by qRT-PCR in a new group of samples, the overexpressed pattern identified for NURR1 (NR4A2) was obtained again, since its expression was higher in r-tPA 100 µg/ml treated cells under OGD compared to control conditions, whereas no changes were detected in r-tPA-untreated cells.

### Example 2

### NURR1 expression in cultured brain endothelial cells

Nurrl was selected as an interesting candidate gene related to the adverse effects of r-tPA treatment because of the highlighted role in several functional pathways and its almost unknown involvement in cerebral ischemia. Hence, its expression and regulation was further tested in brain endothelial cells. In this regard, besides the Nurrl gene expression increase induced by r-tPA under OGD (Figure 2B), our results confirmed that Nurrl protein was also overexpressed after r-tPA treatment in OGD compared to control condition (Figure 2D). Likewise, Nurrl protein was detected in supernatants from cultures treated with r-tPA 100 µg/ml, being highly present under OGD conditions (Figure 2E). In addition, a negative correlation between cell viability and Nurrl gene expression levels was found (p=0.004, r=-0.469) (Figure 2C).

Silencing RNA experiments were designed to test the effect of Nurrl up-regulation in the cytotoxicity induced by r-tPA in OGD-submitted hCMEC/D3 cells. After 48-h transfection, siRNA targeting Nurrl depleted the Nurrl mRNA levels by as much as 64% compared to non-transfected cells and non-targeting siRNA control cells, as shown by qRT-PCR (Figure 3A). Nurrl protein downregulation after siRNA transfection was confirmed by western-blot (Figure 3B).The biological effect of Nurrl silencing was tested after 100 µg/ml r-tPA treatment during 6 h-OGD. Indeed, Nurrl siRNA transfection protected cells from r-tPA cytotoxicity, in comparison with cells transfected with non-targeting siRNA control (p<0.01) (Figure 3C and D). A human immune array was carried out to evaluate the changes in the expression of genes related to inflammation and apoptosis due to Nurrl gene silencing. From 90 genes analyzed, only 50% of them were expressed by hCMEC/D3 cells. Briefly, 9 immune molecules were altered exclusively by r-tPA under OGD conditions (p<0,05), independently of Nurrl gene expression. Knocking down the expression of Nurrl gene resulted in the downregulation of 11 genes under control conditions and, 2 genes, in both OGD and control conditions (p<0.05). Finally, we found 7 downstream targets regulated by Nurrl gene after r-tPA treatment under OGD. On these conditions, Nurrl downregulation prevented the overexpression of some of pro-inflammatory genes induced by r-tPA and OGD.

### Example 3

### NURR1 levels in brain and serum of ischemic stroke patients

First, Nurrl expression was determined in brain tissue from human stroke patients. As shown in Figure 5, both Nurrl gene and protein expression were higher in the infarct core compared to the corresponding contralateral area in r-tPA-treated patients (Nurr1 protein expression; 7.5±3.4 vs 3.5±1.9 pg/ml, p=0.149; Nurrl gene expression; 84±5.4 vs 48.6±13.5 pg/ml, p=0.003, Figure 4B), while no expression changes were found between brain areas of non-treated stroke patients (Figure 4A).

Nurrl protein levels were also analyzed in serum samples from ischemic stroke patients who received thrombolytic treatment within 4.5 h of symptom onset. Serum Nurrl was determined at baseline and after 1h, 12h and 24h after r-tPA treatment. Our study population comprised a total of 28 ischemic stroke patients, 50% were females and mean age was 72.5 years. The ischemic stroke cohort was selected according the presence or not of a hemorrhagic transformation (HT) after receiving thrombolytic treatment (as described in Material and Methods). Thus 50% of the patients suffered an HT, whom 35.7% presented a parenchymal hemorrhage (PH), and, of these 21.4% was symptomatic. The association of baseline Nurrl levels with demographic and risk factors of the study cohort are presented in Table 3.

**Table 3. Demographic factors, vascular risk factors, clinical variables and their influence on NURR1 levels among the studied patients.**

| | | | **Serum NURR1 basal levels (pg/ml)** | | |
|---|---|---|---|---|---|
| | | **Stroke patients (n=28)** | | | |
| | | | **Yes** | **No** | **p** |
| **Sex (Females)** | | 14 (50%) | 349.5 ± 162.0 | 391.2 ± 135.2 | 0.466 |
| **Hypertension** | | 15 (53.6%) | 399.2 ± 158.8 | 337.1 ± 132.7 | 0.276 |
| **Dyslipidemia** | | 8 (28.6%) | 331.3 ± 128.0 | 386.0 ± 155.2 | 0.387 |
| **Diabetes Miellitus** | | 10 (35.7%) | 472.8 ± 129.2 | 313.5 ± 127.8 | **0.004** |
| **Previous stroke** | | 8 (28.6%) | 412.3 ± 152.1 | 353.6 ± 146.8 | 0.352 |
| **Atrial Fibrillation** | | 14 (50%) | 313.7 ± 121.0 | 427.1 ± 154.6 | **0.040** |
| **Hemorrhagic transformation** | | 14 (50%) | 421.7 ± 132.4 | 319.0 ± 132.4 | 0.065 |
| | **Parenchymal hemorrhage** | 10 (35.7%) | 456.0 ± 136.4 | 322.8 ± 134.9 | **0.019** |
| | **Symptomathic** | 6 (21.4%) | 499.2 ± 129.6 | 335.2 ± 134.6 | **0.013** |
| **TOAST** | | | | | |
| | **Cardioembolic** | 16 (57.1%) | 345.4 ± 130.0 | 429.4 ± 149.2 | 0.146 |
| | **Atherothrombotic** | 9 (32.1 %) | 377.5 ± 114.1 | 379.4 ± 157.0 | 0.980 |
| **Ischemic cardiopathy** | | 7 (25%) | 359.6 ± 139.5 | 373.9 ± 153.8 | 0.829 |
| **Anti-Platelet medication** | | 14 (50%) | 367.2 ± 136.0 | 373.5 ± 164.2 | 0.912 |
| **Outcome** | | | | | |
| | **Improvement at 24h** | 15 (53.6%) | 316.8 ± 110.7 | 425.4 ± 171.1 | 0.060 |
| | **Improvement at 48h** | 15 (53.6%) | 298.8 ± 124.0 | 456.9 ± 157.5 | **0.004** |
| **Hospital Death** | | 9 (32.1 %) | 440.2 ± 152.8 | 324.8 ± 129.8 | 0.050 |
| **Rankin 3rd month (mRS >2)** | | 16 (59.3%) | 397.8 ± 163.8 | 313.0 ± 101.9 | 0.141 |
| **Age** ( **years)** | | 72.5 ± 9.4 | R=-0.125 | | 0.525 |
| **Leukocites (U/mm³)** | | 9518.2 ± 2847.5 | R=0.407 | | 0.060 |
| **Platelets (U/mm³)** | | 215450.0 ± 78644.9 | R=0.370 | | 0.090 |
| **Blood Pressure** | | | | | |
| | **Systolic** | 157.4 ± 27 | R=0.048 | | 0.810 |
| | **Diastolic** | 88.5 ± 15.1 | R=0.115 | | 0.562 |
| **Glucose (mg/dl)** | | 141.5 ± 49.6 | R=0.405 | | **0.040** |
| **Baseline Nihss** | | 15.1 ±6.6 | R=0.292 | | 0.132 |

Among them, diabetes mellitus (p=0.004), as well as baseline glucose levels (p=0.04), were the most relevant clinical factors associated with serum baseline Nurrl levels. Regarding the stroke complications associated with thrombolytic treatment, patients who suffered a HT presented slightly higher baseline serum Nurrl levels (421.7±132.4 vs. 319.0±149.3 pg/ml, p=0.065) and even more elevated levels were found in patients who suffered a PH (456.0±136.4 vs. 322.1±134.9 pg/ml, p=0.019). Nevertheless, patients who suffered a symptomatic HT showed the highest baseline Nurrl levels (499.1±129.6 vs. 335.2±134.6 pg/ml, p=0.013) compared to patients with asymptomatic or absence of HT (Figure 5B). ROC curves identified the optimal cut point for Nurrl level association with symptomatic HT, which was 442.48 pg/ml (p=0.007), showing a sensibility of 83.3% and specificity of 81.7%. Indeed, baseline Nurr1 resulted to be the only independent predictor of symptomatic HT in this cohort.

Figure 5A displays the temporal profile of serum Nurrl protein level in ischemic stroke patients according to the type of haemorrhagic transformation after r-tPA administration. Patients who did not suffer an HT or it was asymptomatic presented comparable baseline Nurrl levels than healthy volunteers. Interestingly, serum Nurrl level increased after 1h of r-tPA treatment, particularly in those patients who presented a symptomatic HT after treatment compared with patients without haemorrhagic complications or showing asymptomatic HT (597.1 ± 218.9 vs 346.5 ± 178.1 pg/ml, p=0.008, Figure 5C). After 12 h and 24 h of stroke symptom onset, serum Nurrl concentration decreased in all groups being lower in stroke patients than controls (Figure 5A).

## Claims

1. A method for predicting the risk of suffering symptomatic hemorrhagic transformation in response to the treatment with a thrombolytic agent in an ischemic stroke patient comprising determining the level of Nurr1 in a sample of said patient, wherein elevated level of Nurr1 in said sample with respect to a reference value is indicative that the patient shows high risk of suffering symptomatic hemorrhagic transformation in response to the treatment with a thrombolytic agent.

2. The method according to claim 1 wherein the symptomatic hemorrhagic transformation is a parenchymal hemorrhage.

3. The method according to any of claims 1 or 2 wherein the sample is a biofluid.

4. The method according to claim 3 wherein the biofluid is serum.

5. The method according to claim 4 wherein the serum is obtained before the treatment with the thrombolytic agent or 1 hour after the treatment with the thrombolytic agent.

6. The method according to any of claims 1 to 5 wherein the thrombolytic agent is plasminogen activator.

7. The method according to any of claims 1 to 6 wherein the reference value is 442.48 pg/ml.

8. A composition comprising a Nurr1 inhibitor and a thrombolytic agent.

9. A pharmaceutical preparation comprising a therapeutically effective amount of a composition according to claim 8 and a pharmaceutically acceptable excipient or carrier.

10. An inhibitor of Nurrl for use in the treatment of cytotoxicity associated to the treatment with a thrombolytic agent in a patient suffering ischemic stroke.

11. A composition according to claim 8, a pharmaceutical composition according to claim 9 or an inhibitor of Nurrl for use according to claim 10 wherein the thrombotic agent is a plasminogen activator.

12. A composition according to any of claims 8, 11 or12, an inhibitor of Nurrl for use according to any of claims 10-or 11 or a pharmaceutical composition according to an of claims 9 or 11 wherein the inhibitor of Nurr1 is selected from a compound of table 1.

13. A composition according to any of claims 8, 11 or 12, or a pharmaceutical preparation according to any of claims 9, 11 or 12 for use in the treatment of ischemic stroke.

14. A kit comprising a reagent for detecting the level of Nurr1.

15. Use of the kit according to claim 14 for predicting the risk of suffering symptomatic hemorrhagic transformation in an ischemic stroke patient treated with a thrombolytic agent.
